(19) Europäisches Patentamt
European Patent Office
Office européen des brevets

(11) Numéro de publication: **0 143 688**
**A1**

# (12) DEMANDE DE BREVET EUROPEEN

(21) Numéro de dépôt: **84402152.7**

(22) Date de dépôt: **25.10.84**

(51) Int. Cl.⁴: **C 07 C 121/84**, C 07 C 119/06

(30) Priorité: **25.10.83 FR 8316983**

(43) Date de publication de la demande: **05.06.85**
**Bulletin 85/23**

(84) Etats contractants désignés: **BE CH DE GB IT LI LU NL**

(71) Demandeur: **DESHORS, 38 bis, Rue d'Artois, F-75008 Paris (FR)**

(72) Inventeur: **Berthier, César, 14, Cité Vaneau, F-75007 Paris (FR)**
Inventeur: **Allaigre, Jean-Pierre, 12, rue Danton, F-95460 Ezanville (FR)**
Inventeur: **Desbois, Jacques, 5, rue du Docteur Emile Roux, F-95340 Persan (FR)**

(74) Mandataire: **Ayache, Monique et al, CABINET PLASSERAUD 84, rue d'Amsterdam, F-75009 Paris (FR)**

(54) **Nouveau procédé de préparation de dérivés a-phénylbenzylidèniques d'amino-carboxamides à partir des benzophénones correspondantes et produits intermédiaires.**

(57) On prépare des composés de formule

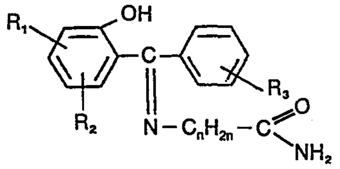

(I)

où $R_1$, $R_2$ et $R_3$ sont H, halogène, $CH_3$ ou $CH_3O$ et $n = 1\text{-}10$ en:
a) formant une base de Schiff entre la benzophénone et l'amino-nitrile correspondants pour obtenir des composés pour la plupart nouveaux, de formule

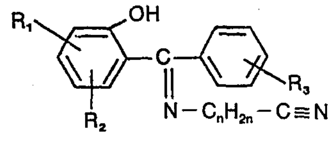

(IV)

et b) on hydrolyse de façon ménagée le nitrile de formule IV, notamment dans un acide, aqueux ou anhydre, en présence de HCl aqueux ou gaz.

Application à la préparation de substances actives de médicaments, notamment le progabide.

EP 0 143 688 A1

Nouveau procédé de préparation de dérivés α-phénylbenzylidèniques d'amino-carboxamides à partir des benzophénones correspondantes et produits intermédiaires.

L'invention a pour objet un nouveau procédé de préparation de dérivés α-phénylbenzylidèniques d'amino-carboxamides répondant à la formule générale :

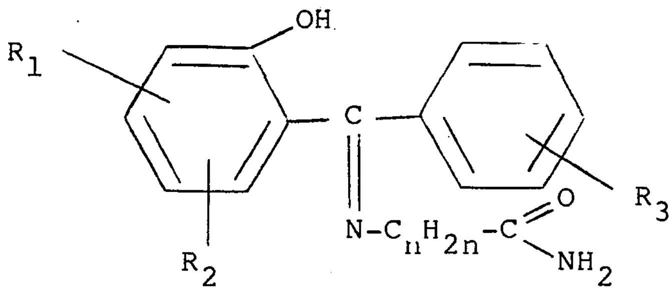

(I)

dans laquelle :

$R_1$, $R_2$ et $R_3$ qui peuvent être identiques ou différents, représentent chacun, indépendamment l'un de l'autre, un atome d'hydrogène, un atome d'halogène, notamment de chlore ou de fluor, ou un groupe méthyle ou méthoxy, et n représente un nombre entier de 1 à 10.

Ces composés qui présentent des propriétés GABA-mimétiques utilisables en thérapeutique sont décrits dans la demande de brevet FR 2 319 338 et la demande de premier certificat d'addition FR 2 358 887 à cette demande de brevet.

On connaît déjà deux procédés de préparation de ces composés à partir des benzophénones correspondantes qui sont des composés connus ou que l'homme du métier peut aisément préparer selon des procédés connus d'obtention des benzophénones.

Selon l'un de ces procédés, on forme une base de Schiff entre la benzophénone et le chlorhydrate d'amino-carboxamide correspondant. Pour mettre en oeuvre ce procédé simple en une étape, on se heurte à des difficultés pour disposer du chlorhydrate d'amino-carboxamide correspondant, notamment du chlorhydrate de γ-aminobutyramide qui est utilisé dans la préparation du progabide ou [α -(4-chlorophényl)-5-fluoro-2-hydroxy-benzylidènyl]-amino-4-butyramide qui est un produit d'un grand intérêt thérapeutique.

Selon l'autre de ces procédés, on forme une base de Schiff entre la benzophénone et l'amino-acide correspondants et on transforme, en deux étapes, le dérivé α-benzylidènique d'amino-acide obtenu en le dérivé d'amino-carboxamide correspondant, par l'intermédiaire du dérivé de chlorure d'acide correspondant. Cette voie d'accès en trois étapes conduit à des rendements relativement peu élevés.

L'invention vise à remédier, au moins en partie, aux inconvénients des procédés de l'art antérieur en fournissant un procédé simple, mis en oeuvre à partir de composés de départ facilement accessibles et conduisant à de bons rendements en produits de formule générale I.

Plus précisément, l'invention a pour objet un procédé de préparation de composés répondant à la formule générale

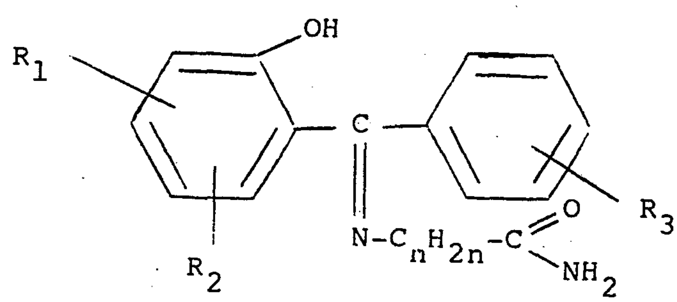

(I)

dans laquelle

$R_1$, $R_2$ et $R_3$ qui peuvent être identiques ou différents, représentent chacun, indépendamment l'un de l'autre, un atome d'hydrogène, un atome d'halogène, notamment de chlore ou de fluor, ou un groupe méthyle ou méthoxy, et

n représente un nombre entier de 1 à 10,

caractérisé en ce que :

a) on forme une base de Schiff entre une benzophénone répondant à la formule générale

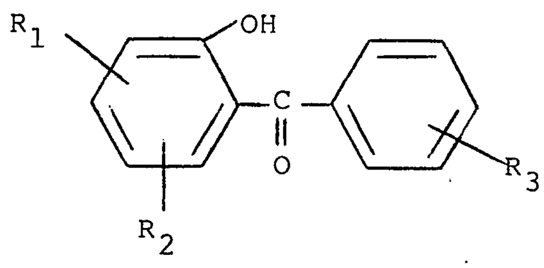

(II)

dans laquelle $R_1$, $R_2$ et $R_3$ ont les significations sus-indiquées, et un aminonitrile répondant à la formule

$$H_2N-C_nH_{2n}-C\equiv N \qquad (III)$$

dans laquelle n a la signification sus-indiquée, ou son halohydrate, de préférence son chlorhydrate, pour obtenir le composé intermédiaire correspondant répondant à la formule

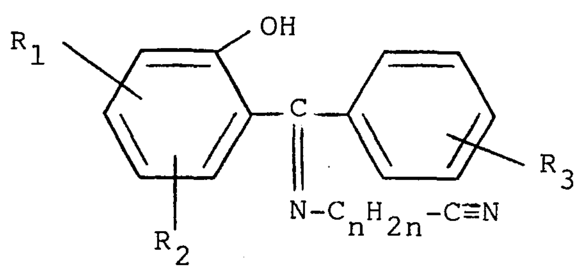

(IV)

dans laquelle $R_1$, $R_2$, $R_3$ et n ont les significations sus-indiquées et

b) on hydrolyse de façon ménagée le nitrile de formule IV en composé de formule I.

La réaction a) entre les benzophénones de formule générale II et les aminonitriles de formule générale III peut être effectuée selon toute technique connue de préparation des bases de Schiff, de préférence dans un solvant polaire tel qu'un alcool comme le méthanol ou l'éthanol, ou un glycol, en présence d'un alcoolate tel que le méthylate ou l'éthylate de sodium, ou en présence de sodium métallique.

En ce qui concerne l'étape b) on entend ici par "on

hydrolyse de façon ménagée" le fait qu'on effectue une hydrolyse dans des conditions suffisamment douces, notamment de température, pour qu'elle ne conduise pas à l'acide correspondant. Cette hydrolyse peut être effectuée dans un acide, aqueux ou anhydre, en présence d'acide chlorhydrique ou de gaz chlorhydrique anhydre.

Selon un mode avantageux de réalisation de l'invention, l'hydrolyse est effectuée dans de l'acide chlorhydrique aqueux concentré, en l'absence de tout autre acide.

Selon un autre mode avantageux de réalisation de l'invention, l'hydrolyse est effectuée dans un acide organique tel que l'acide acétique, l'acide formique ou l'acide propionique, seul ou en mélange, anhydre ou non, en présence d'acide chlorhydrique aqueux ou de gaz chlorhydrique anhydre.

La température de la réaction se situe de préférence dans la gamme de 15 à 55°C.

La durée de la réaction dépend dans une large mesure des conditions opératoires et en particulier de la température choisie. A titre indicatif, elle peut aller de 60 heures à 15°C à environ 10 heures à 55°C.

Les dérivés α-phénylbenzylidèniques d'amino-carboxamides de formule générale I ainsi préparés, peuvent être isolés selon les techniques courantes connues de l'homme du métier telles que l'addition d'un agent désolubilisant comme l'isopropanol ou l'acétone, seuls ou en mélange.

Il peut être avantageux, dans certains cas, d'effectuer une concentration partielle du milieu, sous pression réduite, afin de limiter le volume nécessaire d'agent désolubilisant.

Les composés intermédiaires de formule générale IV sont nouveaux, à l'exception du composé dans lequel $R_1$ = 5-F, $R_2$ = H, $R_3$ = 4'-Cl et n = 3, qui est mentionné dans Chemical Abstracts, vol 97 (1983) référence 97:65869n (J. Chromatogr. 1982, 230(1), 154-61) en tant que produit dérivé du progabide au cours d'une analyse par chromatographie en phase gazeuse.

L'invention vise donc également, à titre de produits nouveaux, les composés de formule générale

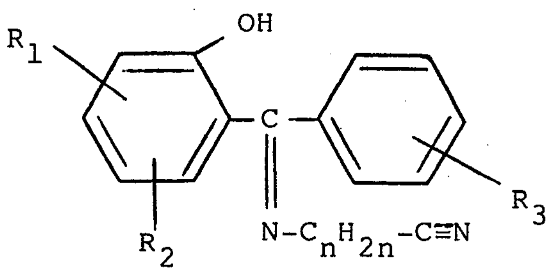

(IV)

dans laquelle :

$R_1$, $R_2$ et $R_3$ qui peuvent être identiques ou différents, représentent chacun, indépendamment l'un de l'autre, un atome d'hydrogène, un atome d'halogène ou un groupe méthyle ou méthoxy et

n représente un nombre entier de 1 à 10, à l'exception du composé dans lequel $R_1$ = 5-F, $R_2$ = H, $R_3$ = 4'-Cl et n = 3.

L'invention sera mieux comprise à l'aide des exemples illustratifs, et en aucune manière limitatifs, qui suivent.

I Exemples de préparation des nitriles de formule IV :

Exemple I.1

N[α(4'-chlorophényl)-5-fluoro-2-hydroxy-benzylidényl]amino-4-butyronitrile

15 g de 4'-chloro-5-fluoro-2-hydroxy-benzophénone et 8 g de chlorhydrate de 4-aminobutyronitrile sont dissous dans 200 ml de méthanol.

On ajoute 6,5 g de méthylate de sodium puis on porte à 50°C. Lorsque la dissolution est complète, on ramène à température ambiante et on maintient ainsi 24 heures. La solution est ensuite concentrée à sec. Le résidu obtenu est repris par 50 ml d'eau, acidifié par de l'acide acétique jusqu'à pH 7 puis extrait au toluène.

Le solvant est évaporé et le résidu est recristallisé dans un mélange heptane/toluène.

On obtient ainsi 12,8 g de cristaux jaunes ;

Rendement : 67 %

Point de fusion : 75°C

Les spectres IR et RMN sont conformes à la structure du produit attendu.

Exemple I.2

N[α(3'-chlorophényl)-5-fluoro-2-hydroxy-benzylidènyl]-amino-2-acétonitrile

7 g de 3'-chloro-5-fluoro-2-hydroxy-benzophénone sont introduits dans un erlenmeyer contenant 2,8 g de chlorhydrate d'aminoacétonitrile et 75 ml d'éthanol. On ajoute 2 g d'éthylate de sodium puis on porte à 40°C pendant 4 heures.

Le milieu est évaporé. Le résidu est repris par 35 ml d'eau et le pH est ajusté à 7 par addition d'acide acétique. La solution est extraite par 30 ml de toluène. Le solvant est évaporé et le résidu est recristallisé dans un mélange heptane-toluène.

On isole ainsi 6,25 g de cristaux ;

Rendement : 72 %

Point de fusion : 90-92°C.

Les spectres IR et RMN sont conformes à la structure du produit attendu.

II Exemples de préparation des amides de formule I

Exemple II.1

N[α(4'-chlorophényl)-5-fluoro-2-hydroxy-benzylidènyl]amino-4-butyramide

31,65 g de [α(4'-chlorophényl)-5-fluoro-2-hydroxy-benzylidènyl]amino-4-butyronitrile sont introduits dans un erlenmeyer contenant 150 ml d'acide formique à 80 %. On ajoute 75 ml d'acide chlorhydrique à 22° Bé (d = 1,19) puis on maintient la solution obtenue à 50°C pendant 12 heures.

On concentre ensuite le milieu, sous pression réduite, au tiers du volume initial.

On désolubilise le produit par addition de 250 ml d'acétone. Les cristaux obtenus sont filtrés puis séchés

en étuve.

On obtient 28,4 g de l'amide recherché ;

Rendement : 85 %

Point de fusion : 133-135°C.

Les spectres IR et RMN sont conformes à la structure du produit recherché.

Exemple II.2

N[α ( 2'-chlorophényl)-5-fluoro-2-hydroxy-benzylidènyl]-amino-4-butyramide

15,75 g de N[α(2'-chlorophényl)-5-fluoro-2-hydroxy-benzylidènyl]amino-4-butyronitrile préparé comme décrit à l'exemple I.1 ou I.2 à partir de 2'-chloro-5-fluoro-2-hydroxy-benzophénone et de 4-aminobutyronitrile, sont mis en suspension dans 200 ml d'acide acétique anhydre. On y fait barbotter un léger courant de gaz chlorhydrique et on maintient le milieu dans ces conditions pendant 10 heures à 45°C. Le courant de gaz chlorhydrique est interrompu et le milieu réactionnel est concentré sous pression réduite à la moitié de son volume initial.

Après addition de 250 ml d'acétone, les cristaux sont filtrés et séchés. On recueille 12 g de cristaux ;

Rendement : ≃ 72 %

Point de fusion : 108-110°C.

Les spectres IR et RMN sont conformes à la structure du produit recherché.

Exemple II.3

N[α(3'chlorophényl)-5-fluoro-2-hydroxy-benzylidènyl]amino-4-butyramide

10,5 g de N[α(3'-chlorophényl)-5-fluoro-2-hydroxy-benzylidènyl]amino-4-butyronitrile préparé comme décrit à l'exemple I.1 ou I.2 à partir de 3'-chloro-5-fluoro-2-hydroxy-benzophénone et de 4-aminobutyronitrile, sont introduits dans un erlenmeyer de 250 ml contenant 50 ml d'acide chlorhydrique à 22° Bé (d = 1,19).

Le milieu est maintenu pendant 35 heures à 30°C. La solution est refroidie entre 0 et 5°C, puis additionnée

de 200 ml d'isopropanol et de 100 ml d'acétone.

Après 5 heures d'agitation à cette température, on recueille 9,5 g de cristaux ;

Rendement : 85 %

Point de fusion : 118-120°C.

Les spectres IR et RMN sont conformes à la structure du produit recherché.

Exemple II.4

N[α(3'-chlorophényl)-5-fluoro-2-hydroxy-benzylidènyl]amino-2-acétamide

14,4 g de N[α(3'-chlorophényl)-5-fluoro-2-hydroxy-benzylidènyl]amino-2-acétonitrile sont dissous dans 35 ml d'acide chlorhydrique à 22° Bé (d = 1,19).

La solution obtenue est portée à 45°C pendant 12 heures.

Après refroidissement et désolubilisation par 250 ml d'acétone, on recueille 11,8 g de cristaux ;

Rendement 77 %.

Point de fusion : 158-160°C.

Les spectres IR et RMN sont conformes à la structure du produit recherché.

REVENDICATIONS

1. Procédé de préparation de composés répondant à la formule générale

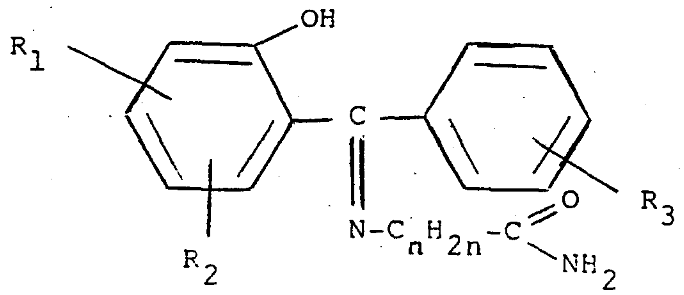

(I)

dans laquelle

$R_1$, $R_2$ et $R_3$ qui peuvent être identiques ou différents, représentent chacun, indépendamment l'un de l'autre, un atome d'hydrogène, un atome d'halogène, notamment de chlore ou de fluor, ou un groupe méthyle ou méthoxy, et n représente un nombre entier de 1 à 10,

caractérisé en ce que :

a) on forme une base de Schiff entre une benzophénone répondant à la formule générale

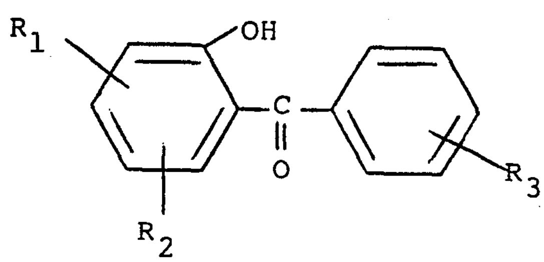

(II)

dans laquelle $R_1$, $R_2$ et $R_3$ ont les significations sus-indiquées, et un aminonitrile répondant à la formule

$$H_2N-C_nH_{2n}-C \equiv N \quad (III)$$

dans laquelle n a la signification sus-indiquée, ou son halohydrate, de préférence son chlorhydrate, pour obtenir le composé intermédiaire correspondant répondant à la formule

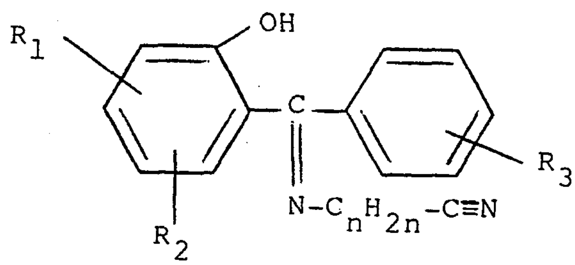

(IV)

dans laquelle $R_1$, $R_2$, $R_3$ et n ont les significations sus-indiquées et

b) on hydrolyse de façon ménagée le nitrile de formule IV en composé de formule I.

2. Procédé selon la revendication 1, caractérisé en ce que la réaction a) entre les benzophénones de formule générale II et les aminonitriles de formule générale III est effectuée dans un solvant polaire tel qu'un alcool comme le méthanol ou l'éthanol, ou un glycol, en présence d'un alcoolate tel que le méthylate ou l'éthylate de sodium, ou en présence de sodium métallique.

3. Procédé selon la revendication 1 ou 2, caractérisé en ce que l'hydrolyse b) est effectuée dans un acide, aqueux ou anhydre, en présence d'acide chlorhydrique ou de gaz chlorhydrique anhydre.

4. Procédé selon la revendication 3, caractérisé en ce que l'hydrolyse est effectuée dans de l'acide chlorhydrique aqueux concentré, en l'absence de tout autre acide.

5. Procédé selon la revendication 3, caractérisé en ce que l'hydrolyse est effectuée dans un acide organique tel que l'acide acétique, l'acide formique ou l'acide propionique, seul ou en mélange, anhydre ou non, en présence d'acide chlorhydrique aqueux ou de gaz chlorhydrique anhydre.

6. Procédé selon l'une des revendications 1 à 5, caractérisé en ce que la température de réaction se situe dans la gamme de 15 à 55°C.

7. Procédé selon la revendication 6, caractérisé en ce que la durée de la réaction est de 60 heures à 15°C à environ 10 heures à 55°C.

8. Procédé selon l'une des revendications 1 à 7, caractérisé en ce que le produit est isolé par addition d'un agent désolubilisant comme l'isopropanol ou l'acétone, seuls ou en mélange.

9. Procédé selon l'une des revendications 1 à 8, caractérisé en ce que pour isoler le produit on effectue une concentration partielle du milieu sous pression réduite puis on ajoute un solvant de désolubilisation.

10. Composés de formule générale

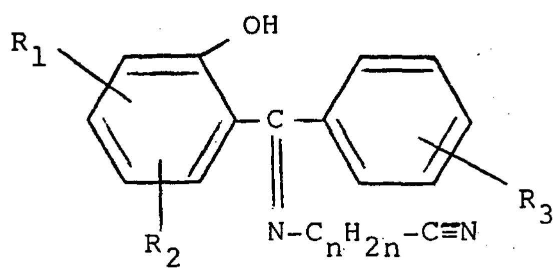

(IV)

dans laquelle :

$R_1$, $R_2$ et $R_3$ qui peuvent être identiques ou différents, représentent chacun, indépendamment l'un de l'autre, un atome d'hydrogène, un atome d'halogène ou un groupe méthyle ou méthoxy et

n représente un nombre entier de 1 à 10, à l'exception du composé dans lequel $R_1$ = 5-F, $R_2$ = H, $R_3$ = 4'-Cl et n = 3.

Office européen des brevets

**RAPPORT DE RECHERCHE EUROPEENNE**

0143688

Numéro de la demande

EP 84 40 2152

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,Y | GB-A-1 529 565 (A. LAWSON)<br>* Pages 1-3; page 7, ligne 31 *<br>--- | 1-9 | C 07 C 121/84<br>C 07 C 119/06 |
| Y,D | FR-A-2 358 887 (SYNTHELABO)<br>* Pages 10-11 *<br>--- | 1 | |
| X | CHEMICAL ABSTRACTS, vol. 97, no. 9, 30 août 1982, page 6, no. 65869n, Columbus, Ohio, US; G. GILLET et al.: "Gas chromatographic method for the determination of progabide (SL 76.002) in biological fluids" & J. CHROMATOGR. 1982, 230(1), 154-161<br>* Abrégé *<br>----- | 10 | |
| | | | DOMAINES TECHNIQUES RECHERCHES (Int. Cl.4) |
| | | | C 07 C 121/00<br>C 07 C 119/00 |

Le présent rapport de recherche a été établi pour toutes les revendications

| Lieu de la recherche | Date d'achèvement de la recherche | Examinateur |
|---|---|---|
| LA HAYE | 01-02-1985 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82